(19) Europäisches Patentamt
European Patent Office
Office européen des brevets

(11) Veröffentlichungsnummer: **0 414 077 B1**

(12) **EUROPÄISCHE PATENTSCHRIFT**

(45) Veröffentlichungstag der Patentschrift: **30.03.94**

(51) Int. Cl.[5]: **C07C 209/18**

(21) Anmeldenummer: **90115443.5**

(22) Anmeldetag: **11.08.90**

# (54) Verfahren zur Herstellung von N,N-Dialkylaninlinen.

(30) Priorität: **25.08.89 DE 3928152**

(43) Veröffentlichungstag der Anmeldung:
**27.02.91 Patentblatt 91/09**

(45) Bekanntmachung des Hinweises auf die Patenterteilung:
**30.03.94 Patentblatt 94/13**

(84) Benannte Vertragsstaaten:
**BE DE FR GB IT**

(56) Entgegenhaltungen:
**EP-A- 0 327 874**
**EP-A- 0 327 875**
**US-A- 4 801 752**

**PATENT ABSTRACTS OF JAPAN, vol. 12, no. 47 (C-475)(2894), 12 Februar 1988, & JP-A-62 195350**

**CHEMICAL ABSTRACTS, vol. 108, no. 3, 18 Januar 1988, Columbus, Ohio, USA; S. HARUHITO ET AL.: "PREPARATION OF N-ALKYLANILINE DERIVATIVES AS INTERMEDIATES FOR DRUGS, AGROCHEMICALS, AND COLOR PHOTOGRAPHY COUPLERS"; & JP-A-62195350**

**CHEMICAL ABSTRACTS, vol. 84, no. 17, 26 April 1976, Columbus, Ohio, USA; T. NOBUO ET AL.: "N-METHYLATION OF ANILINE WITH METHANOL OVER TRANSITION METAL ZEOLITE."**

(73) Patentinhaber: **BAYER AG**

**D-51368 Leverkusen(DE)**

(72) Erfinder: **Buysch, Hans-Josef, Dr.**
**Brandenburger Strasse 28**
**D-4150 Krefeld(DE)**
Erfinder: **Pelster, Heinrich, Dr.**
**Au dem Heidchen 23**
**D-5068 Odenthal(DE)**
Erfinder: **Puppe, Lothar, Dr.**
**Am Weiher 10 a**
**D-5093 Burscheid(DE)**
Erfinder: **Wimmer, Peter, Dr.**
**Bethelstrasse 10**
**D-4150 Krefeld(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die Erfindung betrifft ein Verfahren zur Herstellung von N,N-Dialkylanilinen durch Umsetzung von Anilinen mit niederen Alkoholen bzw. den zugehörigen Ethern bei erhöhter Temperatur und erhöhtem Druck in der Gasphase in Gegenwart von protonenhaltigen Zeolithen des Pentasiltyps.

Es ist bekannt, N,N-dialkylierte Aniline durch Umsetzung von Anilinen mit Alkoholen sowohl in der Flüssig- als auch in der Gasphase in Gegenwart saurer Katalysatoren herzustellen. Im Flüssigphasenverfahren wird die Reaktion in Gegenwart eines flüssigen Katalysators, wie beispielsweise Schwefelsäure, Salzsäure oder Phosphortrichlorid, durchgeführt. Diese Verfahren erfordern eine Arbeitsweise unter Druck. Wegen der stark korrosiven Wirkung der sauren Katalysatoren muß in besonders korrosionsfesten Autoklaven gearbeitet werden. Dennoch kann die Korrosion nicht völlig unterdrückt werden. Darüber hinaus erfordert die Abtrennung und Entsorgung des Katalysators einen beträchtlichen Aufwand.

Die DE-AS 1 031 796 beschreibt die Umsetzung von Anilin mit Alkoholen in der Weise, daß man ein Gemisch von Anilin und überschüssigem Alkohol durch heiße konzentrierte Phosphorsäure leitet. Die Selektivität für N,N-Dialkylverbindungen ist jedoch, insbesondere wenn andere Alkylreste als Methyl eingeführt werden sollen, gering. So erhält man im Falle von Ethyl als Rest bei 220 °C und einem Molverhältnis von Anilin/Ethanol = 1:3 51 % N-Ethylanilin und 15 % N,N-Diethylanilin. Gemäß DE-OS 2 658 728 kann die Ausbeute an N,N-Diethylanilin verbessert werden, wenn man unter Zusatz von aliphatischen Aminen arbeitet; unter sonst analogen Bedingungen erhält man 44 % N-Ethylanilin und 36 % N,N-Diethylanilin. Neben den auch hier vorhandenen Korrosionsproblemen haben solche Verfahren ebenfalls den Nachteil, daß die große Menge Phosphorsäure im Laufe der Zeit als Katalysator unbrauchbar wird und durch frische ersetzt werden muß, so daß auch hier Entsorgungsprobleme auftreten.

In US 4 599 449 ist ein Verfahren zur Gasphasenalkylierung von aromatischen Aminen mit Alkoholen an Übergangsmetalloxid-Katalysatoren beschrieben. Die Selektivität zu N,N-Dialkylanilinen ist jedoch sehr gering. Selbst bei einem 5-fachen Überschuß an Ethanol bleibt der Umsatz des Anilins mit maximal 38 % sehr niedrig und N,N-Diethylanilin wird nur in unzureichender Ausbeute erhalten. Darüber hinaus ist der Anteil an kernalkylierten Nebenprodukten bei diesem Verfahren sehr hoch.

DE-AS 2 335 906 lehrt die N,N-Dialkylierung von Arylaminen mit Alkoholen an Kieselsäure-Katalysatoren, die mit 0,1-20 Gew.-% Phosphorsäure belegt sind. Zur Erzielung guter Selektivitäten ist jedoch ein sehr hoher Überschuß an Alkohol von bis zu 20 Mol-Äquivalenten erforderlich. Dies hat einerseits eine geringe Raum-Zeit-Ausbeute zur Folge, andererseits ist die Abtrennung und Recyclisierung des überschüssigen Alkohols mit einem hohen Destillationsaufwand verbunden. Um eine rasche Desaktivierung des Kontaktes zu vermeiden und eine lange Lebensdauer des Katalysators sicherzustellen, ist es darüber hinaus erforderlich, während der Alkylierung kontinuierlich Phosphorsäure und/oder Phosphorsäurealkylester zuzuführen; ein Teil dieser Phosphorverbindungen wird jedoch stets ausgetragen und verunreinigt das Reaktionsprodukt, wobei aufwendige Abtrennungen erforderlich werden.

Weiterhin ist es bekannt, Zeolithe als Katalysatoren für die Gasphasenalkylierung aromatischer Amine mit Alkoholen einzusetzen. In US 4 801 752 werden Zeolithe vom ZSM 5-Typ mit einem $SiO_2/Al_2O_3$-Verhältnis von 20-700 vorgeschlagen. Die flexible Steuerung der Ausbeute in Richtung des N,N-Dialkylanilins ist jedoch, insbesondere mit anderen Resten als dem Methylrest, nur beschränkt möglich. Unter den günstigsten Bedingungen wird für N,N-Diethylanilin eine maximale molare Selektivität von 10,1 % erzielt. Außerdem müssen bei diesem Verfahren Temperaturen bis über 400 °C angewandt werden. Zusätzlich entsteht ein signifikant hoher Anteil an nicht identifizierten kernalkylierten Nebenprodukten.

In JP 61/35 246 (1986) werden ebenfalls mittelporige Zeolithe als Katalysatoren für die Gasphasenalkylierung von aromatischen Aminen mit Alkoholen beschrieben. Mit diesen Katalysatoren können N-Monoalkylaniline in hoher Selektivität erhalten werden, wobei im Text ausdrücklich hervorgehoben wird, daß die Bildung der N,N-Dialkylaniline an diesen Katalysatoren zurückgedrängt wird. Mittelporige Zeolithe sind also gemäß dieser Publikation nicht geeignet für die Dialkylierung aromatischer Amine.

Es bestand daher weiterhin das Bedürfnis, ein katalytisches Verfahren zur Verfügung zu haben, mit dem eine große Anzahl verschieden substiutierter Aniline in der Gasphase am N-Atom dialkyliert werden können und das die genannten Nachteile nicht aufweist; die Alkylreste sollten insbesondere solche mit mehr als einem C-Atom sein. Die Katalysatoren sollten sich durch einfache Verfügbarkeit, hohe Standzeiten und hohe Aktivitäten auszeichnen und weitgehende Umsätze bei guter Selektivität der N,N-Dialkylierung gewährleisten.

Es wurde nun überraschend gefunden, daß diese Forderungen erfüllt werden, wenn als Katalysatoren protonenhaltige Zeolithe vom Pentasiltyp mit einem $SiO_2/Al_2O_3$-Verhältnis von > 60 eingesetzt werden und die Umsetzung unter erhöhtem Druck durchgeführt wird. Das erfindungsgemäße Verfahren gewährleistet die geforderten hohen Ausbeuten und Umsätze; eine Kernalkylierung tritt nur in geringem Maße auf. Die

verwendeten Katalysatoren weisen hohe Standzeiten auf. Die gefundene Druckabhängigkeit des neuen Verfahrens ist um so überraschender, als sie dem von Le Chatelier formulierten "Prinzip des kleinsten Zwanges" widerspricht. Nach dieser Regel ist eine solche Druckabhängigkeit nicht zu erwarten, da die Reaktion nicht mit einer Volumenkontraktion verbunden ist.

Die Erfindung betrifft daher ein Verfahren zur Herstellung von N,N-Dialkylanilinen durch Umsetzung der zugrundeliegenden Aniline mit niederen Alkoholen oder den zugehörigen Ethern bei erhöhter Temperatur in der Gasphase das dadurch gekennzeichnet ist, daß die Alkylierung in Gegenwart eines protonenhaltigen Zeolith-Katalysators vom Pentasil-Typ mit einem $SiO_2/Al_2O_3$-Verhältnis von > 60, unter einem Druck von 8-30 bar durchgeführt wird.

Das erfindungsgemäße Verfahren wird bei einer Temperatur von 200-400 °C, bevorzugt 250-350 °C, besonders bevorzugt 260-320 °C durchgeführt, Druck und Temperatur sind hierbei so aufeinander abgestimmt, daß sich alle Reaktionspartner in der Gasphase befinden.

Geeignete Zeolithe für das erfindungsgemäße Verfahren sind Pentasile, die Protonen enthalten, also saure Katalysatoren darstellen, und ein $SiO_2/Al_2O_3$-Verhältnis von > 60 aufweisen. Sie können als Kationen ausschließlich Protonen enthalten. Bis zu 80 % der Protonen können jedoch durch andere Kationen substituiert werden. Geeignet hierzu sind beispielsweise die Kationen von Na, K, Mg, Zn, Co, Cu, Ca, Fe, seltene Erden, wie Ce, La und Ga, Sn, Mn, Cr, Ti, Zr, Ta. Bevorzugt werden Pentasile eingesetzt, die weniger als 80 % Metallkationen aufweisen, beispielsweise bis zu 50 %, bevorzugt bis zu 25 %, besonders bevorzugt bis zu 10 %, ganz besonders bevorzugt bis zu 5 %.

Aus der Reihe der Pentasile werden bevorzugt eingesetzt: ZSM 5, ZSM 11, ZSM 8, ZSM 5/ZSM 11 Intermediates, Zeta 1, Zeta 3, ZBM 10, Ultrasil, Ultrazet, TZ-01, NU-4, NU-5, AZ-1. Derartige Zeolithe sind dem Fachmann bekannt und vielfältig beschrieben, beispielsweise in EP 54 386, EP 65 401, EP 34 727, EP 57 016 und EP 113 116, sowie in Russ. J. Phys. Chem. 55 (1981), 1175. Besonders bevorzugt eingesetzte Zeolithe sind: ZSM 5, ZSM 8, ZSM 11 und ZSM 5/ZSM 11 Intermediates; ganz besonders bevorzugt eingesetzt werden Zeolithe vom ZSM 5-Typ.

Das $SiO_2/Al_2O_3$-Verhältnis in den erfindungsgemäß einzusetzenden Pentasiltypen soll größer als 60 sein und beispielsweise 61-2.000, bevorzugt 70-1.500, besonders bevorzugt 80-1.000 betragen. Die Herstellung der zu verwendenden Zeolithe ist in D. W. Breck: Zeolite Molecular Sieves, John Wiley and Sons Inc., New York 1974, beschrieben. Im Zusammenhang mit den genannten Zeolithen vom Pentasiltyp sei weiterhin hingewiesen auf US 3 702 886, US 3 709 979, GB 1 334 243 und EP 18 090.

Damit die Zeolithe in die für den Betrieb eines Gasphasenreaktors günstigere stückige Form gebracht werden können, werden sie mit Bindemitteln verpreßt und granuliert. Als Bindemittel geeignet sind verschiedene Tonerden, Alumosilikate und Aluminumoxide, besonders $\gamma$-$Al_2O_3$, und Siliciumoxid. Für den Fall, daß die Gasphasenreaktion im Wirbelbett durchgeführt wird, eignen sich jedoch auch feinpulvrige Zeolithe.

Zur Durchführung des erfindungsgemäßen Verfahrens werden das am N-Atom zu alkylierende Anilin und das Alkylierungsmittel (niederer Alkohol bzw. zugehörige Ether) verdampft und das verdampfte Gemisch mit dem Zeolith-Katalysator in Kontakt gebracht. Es ist möglich, ein Inertgas, wie Stickstoff, Helium, Wasserdampf, Wasserstoff oder Argon als Trägergas dem Einsatzgemisch zuzusetzen.

Die Katalysatorbelastung LHSV (Liquid Hourly Space Velocity) kann im Bereich von 0,1-4,0 l/l/h, bevorzugt von 0,3-2,0 l/l/h, variiert werden. Die LHSV ist hierbei das Verhältnis des Volumens des flüssigen Anilin/Alkohol-bzw. Anilin/Dialkylether-Gemisches pro Katalysatorvolumen pro Stunde definiert.

Das Reaktionsgemisch wird am Reaktorausgang kondensiert und in bekannter Weise, beispielsweise durch Destillation, aufgetrennt. Nicht umgesetzte Ausgangsstoffe können ebenso wie N-monoalkylierte Produkte in bekannter Weise in die Reaktion zurückgeführt werden.

Im erfindungsgemäßen Verfahren werden gemäß den folgenden Formelgleichungen Aniline der Formel (I) in N,N-dialkylierte Aniline der Formel (II) umgesetzt:

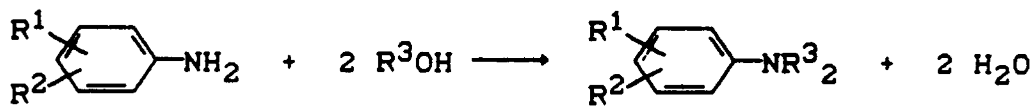

(I) (II)

bzw.

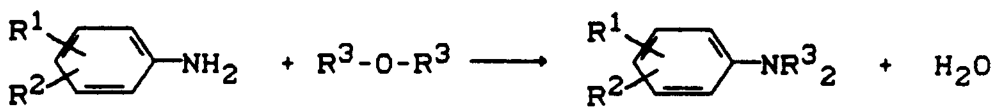

(I) (II)

worin

$R^1$ Wasserstoff, geradkettiges oder verzweigtes $C_1$-$C_8$-Alkyl, Cyclohexyl, Phenyl, geradkettiges oder verzweigtes $C_1$-$C_4$-Alkoxy, Fluor, Chlor, Brom, Cyano oder Nitro bedeutet,

$R^2$ Wasserstoff, geradkettiges oder verzweigtges $C_1$-$C_4$-Alkyl, Fluor, Chlor oder Brom darstellt und

$R^3$ geradkettiges oder verzweigtes $C_1$-$C_4$-Alkyl bedeutet.

Geradkettiges oder verzweigtes $C_1$-$C_8$-Alkyl ist beispielsweise Methyl, Ethyl, Propyl, Isopropyl, Butyl, Isobutyl, tert.-Butyl, die isomeren Hexyle oder Octyle. In bevorzugter Weise seien die genannten $C_1$-$C_4$-Alkylreste genannt. In besonders bevorzugter Weise sei Methyl oder Ethyl genannt.

Geradkettiges oder verzweigtes $C_1$-$C_4$-Alkoxy ist beispielsweise Methoxy, Ethoxy, Propoxy, Isopropoxy, Butoxy oder Isobutoxy, bevorzugt Methoxy oder Ethoxy.

Bevorzugte im erfindungsgemäßen Verfahren umzusetzende Aniline sind solche der Formel

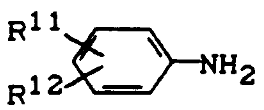

(III),

worin

$R^{11}$ Wasserstoff, Methyl, Ethyl, Propyl, Isopropyl, Cyclohexyl, Methoxy, Ethoxy, Fluor, Chlor, besonders bevorzugt Wasserstoff, Methyl, Chlor, ganz besonders bevorzugt Wasserstoff oder Methyl bedeutet,

$R^{12}$ Wasserstoff, Methyl, Ethyl, Propyl, Isopropyl, Fluor, Chlor, besonders bevorzugt Wasserstoff, Methyl, Chlor, ganz besonders bevorzugt Wasserstoff bedeutet.

Geeignete Aniline für das erfindungsgemäße Verfahren sind beispielsweise Anilin, o-, m-, p-Toluidin, o-, m-, p-Chloranilin, 2,4-, 2,6-, 3,4- oder 3,5-Xylidin, o-, m-, p-Ethylanilin, o-, m-, p-Isopropylanilin und andere; insbesondere seien Anilin und die genannten Toluidine hervorgehoben.

Die zur Alkylierung in den obigen Formeln genannten Alkohole $R^3OH$ bzw. deren zugehörige Ether $R^3$-O-$R^3$ sind dem Fachmann bekannt. Beispielsweise seien genannt: Methanol, Ethanol, Propanol, Isopropanol oder die verschiedenen Butanole bzw. Dimethylether, Diethylether, Dipropylether, Diisopropylether oder die verschiedenen Dibutylether. Grundsätzlich sind auch gemischte Ether erfindungsgemäß umzusetzen, jedoch sind diese wegen der dabei erhältlichen Produktgemische weniger bevorzugt.

Das zu alkylierende Anilin und der Alkohol bzw. der Ether werden im molaren Verhältnis von 1:2-10 (Alkohol) bzw. 1:1-5 (Ether), bevorzugt 1:3-5 bzw. 1:1,5-2,5 eingesetzt. Beim Einsatz eines Ethers wird dieser wie 2 Mol des korrespondierenden Alkohols gerechnet.

N,N-Dialkylaniline sind wichtige industrielle Zwischenprodukte zur Herstellung von Farbstoffen, Pflanzenschutzmitteln und Polymerisations-Co-Katalysatoren (Ullmanns Encyclopädie der technischen Chemie, 4. Auflage, Band 7, S. 572-573); in englisch; 5. Auflage, Band A2, S. 303 ff).

Beispiel 1

In einem senkrecht stehenden Reaktionsrohr von 50 cm Länge und einem Durchmesser von 20 mm wurden 130 ml eines Zeolithen vom Typ H-ZSM 5 mit einem $SiO_2/Al_2O_3$-Verhältnis von 320, der mit 30 % eines handelsüblichen $\gamma$-Aluminiumoxids abgebunden war, mit einem mittleren Korndurchmesser von 1-2 mm eingefüllt. Mittels eines Dreizonenröhrenofens ließ sich der Reaktor thermostatisieren. Die Temperatur in der Katalysatorschüttung konnte mittels eines beweglichen Thermoelementes gemessen werden und betrug 300 °C. Über ein Druckregelventil wurde im Reaktor ein Druck von 10 bar aufrechterhalten. Die Katalyatorbelastung (LHSV) betrug 1,0 l/l/h. Das Reaktionsprodukt wurde auskondensiert und gaschromatographisch analysiert. Umsatz und Selektivität sind in Tabelle 1 zusammengestellt.

Beispiel 2

Es wurde so verfahren, wie in Beispiel 1 beschrieben. Als Katalysator wurde ein H-ZSM 5-Zeolith mit einem $SiO_2/Al_2O_3$-Verhältnis von 272 eingesetzt.

Beispiel 3

Es wurde so verfahren, wie in Beispiel 2 beschrieben. Als Eduktgemisch wurde ein Anilin/Methanol-Gemisch im Molverhältnis 1:2 eingesetzt.

Vergleichsbeispiele 1 bis 3

Die Vergleichsbeispiele wurden unter den den entsprechenden Beispielen analogen Bedingungen, jedoch bei Normaldruck, d.h. bei 1 bar, durchgeführt.

Die Katalysatoren zeichnen sich durch eine hohe Standzeit aus. Nach einer Laufzeit von 300 h waren Umsatz und Ausbeute unverändert.

Aus Tabelle 1 ist klar ersichtlich, daß eine Fahrweise bei 10 bar eine deutliche Umsatzsteigerung, verbunden mit einer ebenso deutlichen Zunahme der Ausbeute an N,N-dialkyliertem Amin zur Folge hat.

**Tabelle 1:** N,N-Dialkylierung von Anilin (Beispiele 1-3 und Vergleichsbeisipele 1-3) an H-ZSM 5 bei 300°C

| Nr. | $SiO_2/Al_2O_3$ | Alkohol | Anilin:Alkohol (molar) | P (bar) | Umsatz (Mol-%) | Gew.-Verhältnis Di-/Mono-Alkyl-anilin |
|---|---|---|---|---|---|---|
| 1 | 326 | EtOH | 1:4 | 10 | 98 | 1.21 |
| Vgl. 1 | 326 | EtOH | 1:4 | 1 | 79 | 0.44 |
| 2 | 272 | EtOH | 1:4 | 10 | 98 | 1.57 |
| Vgl. 2 | 272 | EtOH | 1:4 | 1 | 89 | 0.69 |
| 3 | 272 | MeOH | 1:2 | 10 | 96 | 2.73 |
| Vgl. 3 | 272 | MeOH | 1:2 | 1 | 91 | 1.79 |

## Patentansprüche

1. Verfahren zur Herstellung von N,N-Dialkylanilinen durch Umsetzung der zugrundeliegenden Aniline mit niederen Alkoholen oder den dazugehörigen Ethern bei erhöhter Temperatur in der Gasphase, dadurch

gekennzeichnet, daß die Alkylierung in Gegenwart eines protonenhaltigen Zeolith-Katalysators vom Pentasil-Typ mit einem $SiO_2/Al_2O_3$-Verhältnis von > 60 unter einem Druck von 8-30 bar durchgeführt wird.

**2.** Verfahren nach Anspruch 1, dadurch gekennzeichnet, daß die Alkylierung bei 200-400 °C, bevorzugt 250-350 °C, besonders bevorzugt 260-320 °C durchgeführt wird.

**3.** Verfahren nach Anspruch 1, dadurch gekennzeichnet, daß im Zeolith-Katalysator 0-80 Äquivalent-% der Protonen durch Metallkationen, bevorzugt 0-50 %, besonders bevorzugt 0-25 %, ganz besonders bevorzugt 0-10 %, weiterhin ganz besonders bevorzugt 0-5 %, substiutiert sind.

**4.** Verfahren nach Anspruch 1, dadurch gekennzeichent, daß Zeolith-Katalysatoren der Typen ZSM 5, ZSM 11, ZSM 8, ZSM 5/ZSM 11 Intermediates, Zeta 1, Zeta 3, ZBM 10, Ultrasil, Ultrazet, TZ-01, NU-4, NU-5 und AZ-1, bevorzugt der Typen ZSM 5, ZSM 8, ZSM 11 und ZSM 5/ZSM 11 Intermediates, besonders bevorzugt des Typs ZSM 5 eingesetzt werden.

**5.** Verfahren nach Anspruch 1, dadurch gekennzeichnet, daß das $SiO_2/Al_2O_3$-Verhältnis 61-2.000, bevorzugt 70-1,500, besonders bevorzugt 80-1.000 beträgt.

**6.** Verfahren nach Anspruch 1, dadurch gekennzeichnet, daß die Katalysatorbelastung LHSV 0,1-4,0 1/1/h, bevorzugt 0,3-2,0 1/1/h beträgt.

**7.** Verfahren nach Anspruch 1, dadurch gekennzeichnet, daß Aniline der Formel

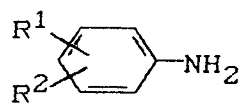

umgesetzt werden, in denen

$R^1$ Wasserstoff, geradkettiges oder verzweigtes $C_1$-$C_8$-Alkyl, Cyclohexyl, Phenyl, geradkettiges oder verzweigtes $C_1$-$C_4$-Alkoxy, Fluor, Chlor, Brom, Cyano oder Nitro bedeutet und

$R^2$ Wasserstoff, geradkettiges oder verzweigtes $C_1$-$C_4$-Alkyl, Fluor, Chlor oder Brom darstellt.

**8.** Verfahren nach Anspruch 1, dadurch gekennzeichnet, daß als Alkohole bzw. Ether solche der Formeln

$R^3OH$ bzw. $R^3$-O-$R^3$

eingesetzt werden, in denen

$R^3$ $C_1$-$C_4$-Alkyl bedeutet.

**9.** Verfahren nach Anspruch 1, dadurch gekennzeichnet, daß das zu alkylierende Anilin und der Alkohol bzw. der Ether im molaren Verhältnis von 1:2-10 (Alkohol) bzw. 1:1-5 (Ether), bevorzugt 1:3-5 bzw. 1:1,5-2,5 eingesetzt werden.

## Claims

**1.** Process for the preparation of N,N-dialkylanilines by reacting the anilines on which they are based with lower alcohols or with the corresponding ethers at an increased temperature in the gas phase, characterized in that the alkylation process is carried out in the presence of a proton-containing zeolite catalyst of the pentasil type having a $SiO_2/Al_2O_3$ ratio of > 60 under a pressure of 8-30 bar.

**2.** Process according to Claim 1, characterized in that the alkylation is carried out at 200-400 °C, preferably 250-350 °C, and particularly preferably 260-320 °C.

**3.** Process according to Claim 1, characterized in that 0-80 equivalent percent, preferably 0-50%, particularly preferably 0-25% and very particularly preferably 0-10%, and especially preferably 0-5%, of

the protons in the zeolite catalyst are substituted by metal cations.

4. Process according to Claim 1, characterized in that zeolite catalysts of the types ZSM 5, ZSM 11, ZSM 8, ZSM 5/ZSM 11 intermediates, Zeta 1, Zeta 3, ZBM 10, Ultrasil, Ultraset, TZ-01, NU-4, NU-5 and AZ-1, preferably of the types ZSM 5, ZSM 8, ZSM 11 and ZSM 5/ZSM 11 Intermediates, and particularly preferably of the ZSM 5 type, are employed.

5. Process according to Claim 1, characterized in that the $SiO_2/Al_2O_3$ ratio is 61 : 2,000, preferably 70 : 1,500, and particularly preferably 80 : 1,000.

6. Process according to Claim 1, characterized in that the load of the catalyst LHSV is 0.1-4.0 l/l/h, preferably 0.3-2.0 l/l/h.

7. Process according to Claim 1, characterized in that anilines of the formula

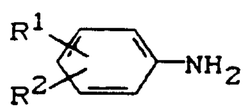

are reacted in which

$R^1$ denotes hydrogen, straight-chain or branched $C_1$-$C_8$-alkyl, cyclohexyl, phenyl, straight-chain or branched $C_1$-$C_4$-alkoxy, fluorine, chlorine, bromine, cyano or nitro, and

$R^2$ represents hydrogen, straight-chain or branched $C_1$-$C_4$-alkyl, fluorine, chlorine or bromine.

8. Process according to Claim 1, characterized in that the alcohols or ethers employed are those of the formulae

$R^3OH$ and $R^3$-O-$R^3$,

respectively, in which

$R^3$ denotes $C_1$-$C_4$-alkyl.

9. Process according to Claim 1, characterized in that the aniline to be alkylated and the alcohol or the ether are employed in a molar ratio of 1:2-10 (alcohol) or 1:1-5 (ether), respectively, preferably 1:3-5 or 1:1.5-2.5, respectively.

**Revendications**

1. Procédé de production de N,N-dialkylanilines par réaction des anilines de base avec des alcools inférieurs ou les éthers correspondants, à température élevée en phase gazeuse, caractérisé en ce que l'alkylation est conduite en présence d'un catalyseur zéolitique contenant des protons du type Pentasil avec un rapport $SiO_2/Al_2O_3$ supérieur à 60, sous une pression de 8 à 30 bars.

2. Procédé suivant la revendication 1, caractérisé en ce qu'on conduit l'alkylation à 200-400 °C, de préférence à 250-350 °C, notamment à 260-320 °C.

3. Procédé suivant la revendication 1, caractérisé en ce que dans le catalyseur zéolitique, une proportion de 0 à 80 équivalents pour cent des protons est remplacée par des ions métalliques, avantageusement une proportion de 0 à 50 %, mieux encore une proportion de 25 %, très avantageusement une proportion de 0 à 10 % et notamment une proportion de 0 à 5 %.

4. Procédé suivant la revendication 1, caractérisé en ce qu'on utilise des catalyseurs zéolitiques des types ZSM 5, ZSM 11, ZSM 8, ZSM 5/ZSM 11 intermédiaires, Zéta 1, Zéta 3, ZBM 10, Ultrasil, Ultrazet, TZ-01, NU-4, NU-5 et AZ-1, de préférence des types ZSM 5, ZSM 8, ZSM 11 et ZSM 5/ZSM 11 intermédiaire, notamment du type ZSM 5.

**5.** Procédé suivant la revendication 1, caractérisé en ce que le rapport $SiO_2/Al_2O_3$ a une valeur de 61-2000, mieux encore de 70-1500, notamment de 80-1000.

**6.** Procédé suivant la revendication 1, caractérisé en ce que la charge du catatyseur s'élève à une VSHL de 0,1-4,0 l/l/h, de préférence de 0,3-2,0 l/l/h.

**7.** Procédé suivant la revendication 1, caractérisé en ce qu'on fait réagir des anilines de formule

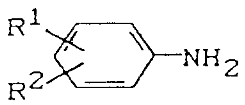

dans lesquelles

$R^1$ représente l'hydrogène, un groupe alkyle en $C_1$ à $C_8$ à chaîne droite ou ramifiée, cyclohexyle, phényle, alkoxy en $C_1$ à $C_4$ à chaîne droite ou ramifiée, du fluor, du chlore, du brome, un groupe cyano ou nitro et

$R^2$ représente de l'hydrogène, un groupe alkyle en $C_1$ à $C_4$ à chaîne droite ou ramifiée, du fluor, du chlore ou du brome.

**8.** Procédé suivant la revendication 1, caractérisé en ce qu'on utilise comme alcools ou éthers des composés de formules

$R^3OH$ et respectivement $R^3$-O-$R^3$

dans lesquelles

$R^3$ est un groupe alkyle en $C_1$ à $C_4$.

**9.** Procédé suivant la revendication 1, caractérisé en ce que l'aniline à alkyler et l'alcool ou l'éther sont utilisés dans un rapport molaire de 1:2-10 (alcool) ou respectivement de 1:1-5 (éther), de préférence 1:3-5 ou respectivemenet 1:1,5-2,5.